# EUROPÄISCHE PATENTSCHRIFT

(11) **EP 4 464 282 B1**
(45) Veröffentlichungstag und Bekanntmachung des Hinweises auf die Patenterteilung: **11.11.2026**
(21) Anmeldenummer: 24171369.2
(22) Anmeldetag: 19.04.2024
(51) Int. Cl.: A61F 2/58, B25J 15/00

(54) **DISTALES FINGERPROTHESENMODUL FÜR EINE MODULARE FINGERPROTHESE**
DISTAL FINGER PROSTHESIS MODULE FOR A MODULAR FINGER PROSTHESIS
MODULE DE PROTHÈSE DE DOIGT DISTAL POUR PROTHÈSE DE DOIGT MODULAIRE

(30) Priorität: 15.05.2023 DE 102023002140
(43) Veröffentlichungstag der Anmeldung: 20.11.2024
(73) Patentinhaber: Lang, Daniel, 71691 Freiberg a.N. Baden-Württemberg (DE)
(72) Erfinder: Lang, Daniel, 71691 Freiberg a.N. Baden-Württemberg (DE)

(56) Entgegenhaltungen:
- IT-A1- 202100 007 337
- US-A1- 2005 043 822
- US-A1- 2010 042 229
- US-A1- 2013 345 828
- US-A1- 2021 145 146

## Beschreibung

Die vorliegende Erfindung betrifft ein distales Fingerprothesenmodul für eine modulare Fingerprothese sowie eine Funktionseinheit für ein derartiges distales Fingerprothesenmodul. Die vorliegende Erfindung betrifft ferner eine distale Fingerprothesenmoduleinheit für eine modulare Fingerprothese sowie eine entsprechende modulare Fingerprothese.

### Stand der Technik

Der Körper ist das beste Werkzeug, welches der menschliche Verstand besitzt. Zu diesem Werkzeug zählen auch unsere Finger.

Die CA 2 975 446 A1 offenbart einen prothetischen Finger mit einem Touchscreen-Mechanismus, um es dem Benutzer zu ermöglichen, den prothetischen Finger zur Bedienung kapazitiver Touchscreens zu verwenden, die auf den Eigenstrom des Körpers reagieren. Dokument US 2005/043822 A1 und US 2010/042229 A1 beschreiben Fingerprothesen mit zusätzlichen Funktionseinheiten.

### Beschreibung der Erfindung

Vor diesem Hintergrund wird mit dem hier vorgestellten Ansatz ein distales Fingerprothesenmodul für eine modulare Fingerprothese vorgestellt, mit
- einem Verbindungselement zur lösbaren Verbindung des Fingerprothesenmoduls mit der modularen Fingerprothese bzw. einem proximalen Verbindungsmodul der modularen Fingerprothese, und
- einer Aufnahmeeinheit in einem Innenbereich und/oder an einer Oberseite des distalen Fingerprothesenmoduls zur lösbaren Aufnahme einer Funktionseinheit zur Bereitstellung einer zusätzlichen Funktion für eine die modulare Fingerprothese tragende Person.

Ferner wird eine Funktionseinheit für ein distales Fingerprothesenmodul der vorangehend beschriebenen Art vorgestellt, welche ausgebildet ist, in der Aufnahmeeinheit des distalen Fingerprothesenmoduls lösbar aufgenommen zu werden.

### Des Weiteren wird eine distale Fingerprothesenmoduleinheit für eine modulare

Fingerprothese vorgestellt, mit
- einem distalen Fingerprothesenmodul der vorangehend beschriebenen Art und
- einer Funktionseinheit der vorangehend beschriebenen Art, welche in der Aufnahmeeinheit des distalen Fingerprothesenmoduls lösbar aufgenommen ist.

Außerdem wird eine entsprechende modulare Fingerprothese vorgestellt, mit
- einem distalen Fingerprothesenmodul der vorangehend beschriebenen Art oder
- einer Fingerprothesenmoduleinheit der vorangehend beschriebenen Art, welches/welche mittels des Verbindungselements mit der modularen Fingerprothese bzw. einem proximalen Verbindungsmodul der modularen Fingerprothese lösbar verbunden ist.

Die vorliegende Erfindung stellt ein einfach, bspw. mittels 3D-Druck herstellbares, und multifunktionales distales Fingerprothesenmodul sowie eine modulare Fingerprothese bereit, welches bedarfsgerecht in Hinblick auf seine Funktionen sowie ggf. sein Erscheinungsbild für eine die modulare Fingerprothese tragende Person leicht und schnell anpassbar bzw. personalisierbar ist.

Unter einer modularen Fingerprothese soll im Rahmen der vorliegenden Erfindung eine Fingerprothese verstanden werden, welche einen modularisierten bzw. bausteinförmigen Aufbau aufweist, wobei die Fingerprothesenmodule entlang definierter Verbindungs- bzw. Schnittstellen lösbar zusammengesetzt sind. Demnach ist die modulare Fingerprothese sehr einfach und vielfältig durch Austausch eines jeweiligen Fingerprothesenmoduls an die körperlichen Abmessungen der tragenden Person anpassbar. Der Austausch kann hierbei insbesondere ohne Werkzeug erfolgen.

Die modulare Fingerprothese kann bspw. ein proximales Verbindungsmodul aufweisen, welches mit einer menschlichen Hand, insbesondere einem entsprechenden menschlichen Fingerglied verbindbar bzw. an dieser/diesem fixierbar ist. Mit dem proximalen Verbindungsmodul kann wiederum das distale Fingerprothesenmodul verbindbar bzw. koppelbar sein, um die modulare Fingerprothese auszubilden. Demnach fungiert das proximale Verbindungsmodul als Verbindungselement des gesunden Fingerglieds mit dem künstlichen distalen Fingerprothesenmodul.

Das proximale Verbindungsmodul kann in distaler Richtung gesehen ein Erstmodul bzw. Ringmodul, ein Zwischenmodul und ein Endmodul aufweisen. Das Ringmodul, das Zwischenmodul und das Endmodul können hierbei derart über Schnüre bzw. Drähte miteinander gekoppelt sein, dass eine entsprechende Bewegungsübertragung erfolgt, sodass eine Griff-/Schließbewegung und eine Öffnungsbewegung durchführbar sind. Das Erstmodul bzw. Ringmodul kann über das entsprechende menschliche Fingerglied aufschiebbar sein, um das proximale Verbindungsmodul mit diesem zu verbinden. Das Zwischenmodul kann jeweils gelenkig mit dem Erstmodul und dem Endmodul verbunden sein. Das Endmodul kann einen komplementären Verbindungsabschnitt für die lösbare Verbindung bzw. Kopplung mit dem distalen Fingerprothesenmodul aufweisen.

Der komplementäre Verbindungsabschnitt kann für eine lösbare formschlüssige Verbindung mit dem distalen Fingerprothesenmodul
- zumindest eine nutenförmige Vertiefung bzw. Sacklochnut aufweisen, in die zumindest ein Vorsprung des distalen Fingerprothesenmoduls, insbesondere entgegen einer bestimmungsgemäßen Griffrichtung des distalen Fingerprothesenmoduls einschiebbar ist, und/oder
- zumindest einen Vorsprung aufweisen, welcher in zumindest eine nutenförmige Vertiefung bzw. Sacklochnut des distalen Fingerprothesenmoduls, insbesondere entgegen einer/der bestimmungsgemäßen Griffrichtung des distalen Fingerprothesenmoduls einschiebbar ist.

Das distale Fingerprothesenmodul ist als distales Fingerprothesenglied für den Einsatz in einer entsprechenden modularen Fingerprothese ausgebildet. D.h., mit anderen Worten, dass das distale Fingerprothesenmodul ausgebildet ist, mit einem entsprechend ausgebildeten proximalen Verbindungsmodul einer modularen Fingerprothese verbunden bzw. gekoppelt zu werden, um das fehlende menschliche Fingerglied zu ersetzen bzw. als fehlendes oder zusätzliches menschliches Fingerglied zu fungieren.

Hierbei weist das distale Fingerprothesenmodul zum einen ein Verbindungselement auf. Das Verbindungselement ist ausgebildet, das distale Fingerprothesenmodul lösbar mit der modularen Fingerprothese bzw. einem komplementären Verbindungsabschnitt der modularen Fingerprothese zu verbinden.

Vorteilhafterweise kann das Verbindungselement ausgebildet sein, eine lösbare formschlüssige Verbindung, insbesondere lösbare formschlüssige Rastverbindung mit einem komplementären Verbindungsabschnitt der modularen Fingerprothese einzugeben. Hierbei kann das Verbindungselement zur lösbaren formschlüssigen Verbindung mit der modularen Fingerprothese
- zumindest einen Vorsprung aufweisen, welcher in zumindest eine nutenförmige Vertiefung des komplementären Verbindungsabschnitts der modularen Fingerprothese, insbesondere entgegen einer bestimmungsgemäßen Griffrichtung des distalen Fingerprothesenmoduls einschiebbar ist, und/oder
- zumindest eine nutenförmige Vertiefung aufweisen, in die zumindest ein Vorsprung des komplementären Verbindungsabschnitts der modularen Fingerprothese, insbesondere entgegen einer/der bestimmungsgemäßen Griffrichtung des distalen Fingerprothesenmoduls einschiebbar ist.

Durch diese Maßnahme kann eine einfache lösbare Steckverbindung zwischen dem distalen Fingerprothesenmodul und der modularen Fingerprothese bzw. dem Verbindungsmodul der modularen Fingerprothese bereitgestellt werden, welche sich ferner bei einer Griffbewegung selbst in Position bringt, wobei aufgrund der Druckrichtung einem versehentlichen Lösen der Verbindung bzw. Steckverbindung entgegengewirkt wird.

Zum anderen weist das distale Fingerprothesenmodul eine Aufnahmeeinheit auf, welche ausgebildet ist, eine Funktionseinheit zur Bereitstellung einer zusätzlichen Funktion für eine die modulare Fingerprothese tragende Person lösbar aufzunehmen. D.h., mit anderen Worten, dass die Aufnahmeeinheit ausgebildet ist, eine entsprechend ausgebildete Funktionseinheit aufzunehmen und wieder freizugeben, sodass diese einfach auswechselbar ist. Die Aufnahmeeinheit ist hierbei in einem Innenbereich und/oder an einer Oberseite des distalen Fingerprothesenmoduls angeordnet. Demnach kann die Aufnahmeeinheit in einem größtenteils umschlossenen oder vollständig umschlossenen Innenbereich und/oder an einer Oberseite, d.h. einer Fingernagelseite bzw. Handrückseite, angeordnet sein.

Unter einer zusätzlichen Funktion soll hierbei eine zusätzlich zur Grifffunktion des distalen Fingerprothesenmoduls bereitgestellte Funktion verstanden werden. Hierbei handelt es sich insbesondere um eine technische Funktion bzw. ein zusätzliches Werkzeug, wie nachfolgend näher erläutert wird.

Das distale Fingerprothesenmodul kann einen Hauptkörper aufweisen. Der Hauptkörper kann einstückig bzw. einteilig oder mehrteilig aufgebaut sein. Der Hauptkörper kann bspw. ein Oberteil, welches als Nagelbett fungiert, und ein Unterteil, welches als Fingerbeere fungiert, aufweisen. Das Verbindungselement kann an dem Hauptkörper, insbesondere an einem proximalen Endbereich des Hauptkörpers angeordnet sein. Die Aufnahmeeinheit kann in dem Innenbereich des Hauptkörpers und/oder an einer Oberseite des Hauptkörpers angeordnet sein. Bei der mehrteiligen Ausführungsform kann der Innenbereich zwischen dem Oberteil und dem Unterteil angeordnet sein und/oder die Oberseite eine Oberseite des Oberteils sein.

Vorteilhafterweise kann die Aufnahmeeinheit ausgebildet sein, eine lösbare formschlüssige Verbindung, insbesondere eine lösbare formschlüssige Rastverbindung mit einem komplementären Verbindungsabschnitt der Funktionseinheit einzugehen.

Hierbei kann die Aufnahmeeinheit zur lösbaren Aufnahme der Funktionseinheit, insbesondere zur lösbaren formschlüssigen Verbindung mit der Funktionseinheit bevorzugt
- zumindest einen Aufnahmeraum in dem Innenbereich aufweisen, in den die Funktionseinheit, insbesondere vollständig aufnehmbar ist, und/oder
- zumindest eine nutenförmige Vertiefung an der Oberseite aufweisen, in die zumindest ein Vorsprung des komplementären Verbindungsabschnitts der Funktionseinheit einbringbar, insbesondere proximal einschiebbar ist, und/oder
- zumindest einen Vorsprung an der Oberseite aufweisen, welcher in zumindest eine nutenförmige Vertiefung des komplementären Verbindungsabschnitts der Funktionseinheit einbringbar, insbesondere proximal einschiebbar ist.

Demnach kann die Aufnahmeeinheit einen Aufnahmeraum bzw. eine Aufnahmekammer im Innenbereich aufweisen oder als ein Aufnahmeraum bzw. eine Aufnahmekammer im Innenbereich ausgebildet sein. Bevorzugt kann der Aufnahmeraum eine, insbesondere schließbare Öffnung zum Ein- und Ausbringen, insbesondere Ein- und Ausschieben der Funktionseinheit aufweisen. Hierfür kann das distale Fingerprothesenmodul einen entsprechend ausgebildeten Deckel aufweisen. Alternativ oder zusätzlich kann die Aufnahmeeinheit einen Aufnahmebereich an der Oberseite aufweisen oder als ein Aufnahmebereich an der Oberseite ausgebildet sein.

Analog kann die Funktionseinheit vorteilhafterweise einen komplementären Verbindungsabschnitt aufweisen, welcher ausgebildet ist, eine lösbare formschlüssige Verbindung, insbesondere lösbare formschlüssige Rastverbindung mit der Aufnahmeeinheit einzugehen. Hierbei kann der komplementäre Verbindungsabschnitt zur lösbaren formschlüssigen Verbindung mit der Aufnahmeeinheit des distalen Fingerprothesenmoduls
- zumindest eine nutenförmige Vertiefung, insbesondere an einer Unterseite aufweisen, in die zumindest einen Vorsprung der Aufnahmeeinheit einbringbar, insbesondere proximal einschiebbar ist, und/oder
- zumindest einen Vorsprung, insbesondere an einer/der Unterseite aufweisen, welcher in zumindest eine nutenförmige Vertiefung der Aufnahmeeinheit einbringbar, insbesondere proximal einschiebbar ist.

Es ist ferner von Vorteil, wenn die Aufnahmeeinheit zur lösbaren formschlüssigen Rastverbindung mit der Funktionseinheit
- zumindest einen elastisch federnden Rasthaken aufweist, welcher in zumindest einer Rastvertiefung des komplementären Verbindungsabschnitts der Funktionseinheit einrastbar ist, und/oder
- zumindest eine Rastvertiefung aufweist, in die zumindest ein elastisch federnder Rasthaken des komplementären Verbindungsabschnitts der Funktionseinheit einrastbar ist.

Hierbei ist es vorteilhaft, wenn zur Freigabe der Rastverbindung
- die Aufnahmeeinheit zumindest ein Entriegelungselement, insbesondere einen Druckknopf aufweist, mittels dessen der elastisch federnde Rasthaken betätigbar ist, und/oder
- die zumindest eine Rastvertiefung derart ausgebildet ist, dass von außen Druck auf den eingerasteten elastisch federnden Rasthaken der Funktionseinheit ausübbar ist,
um die eingerastete Funktionseinheit freizugeben, insbesondere wobei das Entriegelungselement und/oder die Rastvertiefung an einem proximalen Endbereich der Aufnahmeeinheit angeordnet ist.

Durch diese Maßnahme kann die Funktionseinheit sehr einfach einrastend eingesteckt und durch eine Druckbetätigung wieder freigegeben werden.

Analog kann der komplementäre Verbindungsabschnitt der Funktionseinheit vorteilhafterweise zur lösbaren formschlüssigen Rastverbindung mit der Aufnahmeeinheit des distalen Fingerprothesenmoduls
- zumindest eine Rastvertiefung aufweisen, in die zumindest ein elastisch federnder Rasthaken der Aufnahmeeinheit einrastbar ist, und/oder
- zumindest einen elastisch federnden Rasthaken aufweisen, welcher in zumindest einer Rastvertiefung der Aufnahmeeinheit einrastbar ist, insbesondere wobei durch Druckausübung auf den eingerasteten elastisch federnden Rasthaken die eingerastete Funktionseinheit freigebbar ist.

Hierbei ist es ferner vorteilhaft, wenn die Funktionseinheit zumindest eine der folgenden Einheiten aufweist oder als zumindest eine der folgenden Einheiten ausgebildet ist:
- Lichteinheit, insbesondere LED-Beleuchtungseinheit und/oder Laserpointer;
- Datenspeichereinheit, insbesondere USB-Stick;
- Energiespeichereinheit, insbesondere Powerbank;
- Erwärmungseinheit, insbesondere Feuerzeug;
- Schreibeinheit, insbesondere Minenhalter für eine Schreibmine oder mit einer Schreibmine;
- Werkzeugeinheit, insbesondere Werkzeughalter für ein Werkzeug und/oder mit einem Werkzeug;
- Magneteinheit, insbesondere für ein berührungsempfindliches Tastfeld bzw. Touchpad;
- Zwille;
- Zerstäubungseinheit, insbesondere Sprüheinheit;
- Identifikationseinheit, insbesondere RFID-Chip.

Das Werkzeug der Werkzeugeinheit kann hierbei bspw. ein Schraubbit, ein Messer, eine Pinzette oder ein Skalpell sein.

Demnach ist es von Vorteil, wenn bei der distalen Fingerprothesenmoduleinheit die Aufnahmeeinheit in dem Innenbereich des distalen Fingerprothesenmoduls angeordnet ist und in ihr die vorangehend beschriebene Funktionseinheit lösbar aufgenommen ist. Die vorangehend beschriebene Funktionseinheit kann jedoch auch ohne Weiteres ausgebildet sein, in der Aufnahmeeinheit an der Oberseite des distalen Fingerprothesenmoduls aufgenommen zu werden.

Alternativ oder zusätzlich kann die Funktionseinheit bevorzugt zumindest einen künstlichen Fingernagel aufweisen oder als zumindest ein künstlicher Fingernagel ausgebildet sein. Der Fingernagel kann im täglichen Leben eine Vielzahl von technischen und optischen Funktionen erfüllen. Somit kann er durch diese Maßnahme bedarfsgerecht angepasst bzw. personalisiert werden, bspw. in Hinblick auf Größe, insbesondere Länge; Dicke; Form, insbesondere Kontur; Material optische Ausgestaltung, insbesondere Farbe und Verzierung.

Demnach ist es von Vorteil, wenn bei der distalen Fingerprothesenmoduleinheit die Aufnahmeeinheit an der Oberseite des distalen Fingerprothesenmoduls angeordnet ist und in ihr ein künstlicher Fingernagel als Funktionseinheit aufgenommen ist.

Bevorzugt weist die distale Fingerprothesenmoduleinheit eine Aufnahmeeinheit auf, welche in einem Innenbereich und an einer Oberseite des distalen Fingerprothesenmoduls angeordnet ist, wobei in dem Innenbereich die oben beschriebene Funktionseinheit und an der Oberseite ein künstlicher Fingernagel als Funktionseinheit lösbar aufgenommen sind.

### Zeichnungen

Die Erfindung wird nachstehend anhand der beigefügten Zeichnungen beispielhaft näher erläutert. Es zeigen:
- Figur 1: eine perspektivische Darstellung einer erfindungsgemäßen modularen Fingerprothese mit einer erfindungsgemäßen distalen Fingerprothesenmoduleinheit;
- Figur 2: eine perspektivische Darstellung des Ringmoduls der modularen Fingerprothese aus Fig. 1;
- Figur 3: eine perspektivische Darstellung des Zwischenmoduls der modularen Fingerprothese aus Fig. 1;
- Figur 4: eine perspektivische Darstellung des Endmoduls der modularen Fingerprothese aus Fig. 1;
- Figur 5: eine perspektivische Darstellung der erfindungsgemäßen distalen Fingerprothesenmoduleinheit aus Fig. 1;
- Figur 6: eine perspektivische Darstellung eines Unterteils des distalen Fingerprothesenmoduls aus Fig. 1 und 5;
- Figur 7: eine perspektivische Darstellung eines Oberteils des distalen Fingerprothesenmoduls aus Fig. 1 und 5;
- Figur 8: eine perspektivische Darstellung einer als künstlicher Nagel ausgebildeten Funktionseinheit aus Fig. 1 und 5;
- Figur 9a-b: ein weiteres Ausführungsbeispiel eines erfindungsgemäßen distalen Fingerprothesenmoduls;
- Figur 10a-c: eine als Schreibeinheit ausgebildete erfindungsgemäße Funktionseinheit für das distale Fingerprothesenmodul aus Fig. 9a-b;
- Figur 11a-c: eine als Werkzeugeinheit ausgebildete erfindungsgemäße Funktionseinheit für das distale Fingerprothesenmodul aus Fig. 9a-b;
- Figur 12a-b: eine als Feuerzeug ausgebildete erfindungsgemäße Funktionseinheit für das distale Fingerprothesenmodul aus Fig. 9a-b.

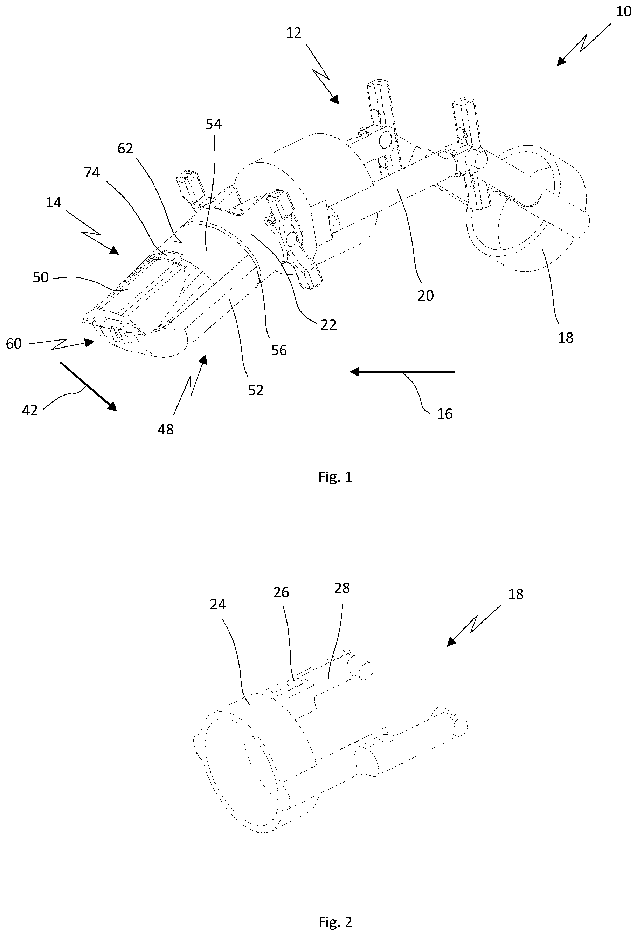

Fig. 1 zeigt eine perspektivische Darstellung einer erfindungsgemäßen modularen Fingerprothese, welche in ihrer Gesamtheit mit der Bezugsziffer 10 versehen ist.

Die modulare Fingerprothese 10 weist ein proximales Verbindungsmodul 12 und eine mit diesem lösbar verbundene erfindungsgemäße distale Fingerprothesenmoduleinheit 14 auf.

Das proximale Verbindungsmodul 12 ist mit einem entsprechenden (nicht dargestellten) menschlichen Fingerglied verbindbar bzw. an diesem fixierbar. Demnach fungiert das proximale Verbindungsmodul 12 als Verbindungelement des gesunden Fingerglieds mit der künstlichen distalen Fingerprothesenmoduleinheit 14. Hierfür weist das proximale Verbindungsmodul 12 in distaler Richtung 16 ein Erstmodul bzw. Ringmodul 18, ein Zwischenmodul 20 und ein Endmodul 22 auf. Das Ringmodul 18, das Zwischenmodul 20 und das Endmodul 22 sind hierbei derart über (nicht dargestellte) Schnüre bzw. Drähte miteinander gekoppelt, dass eine entsprechende Bewegungsübertragung erfolgt, sodass eine Griff-/Schließbewegung und eine Öffnungsbewegung durchführbar sind.

Das Ringmodul 16 ist in Fig. 2 näher dargestellt. Das Ringmodul 16 weist einen Ring 24 auf, welcher über das entsprechende menschliche Fingerglied aufschiebbar ist, um das proximale Verbindungsmodul 12 mit diesem zu verbinden. Das Ringmodul 16 weist ferner Öffnungen 26 an Stegen 28 zur proximalen Fixierung der Schnüre auf.

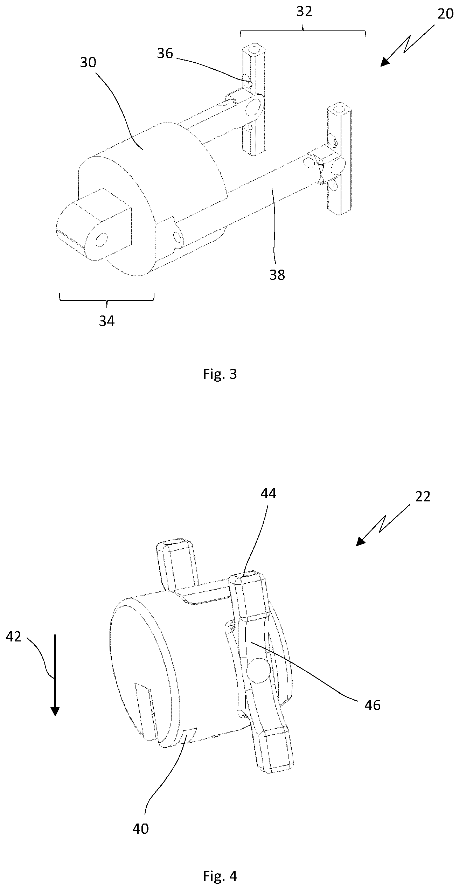

Das Zwischenmodul 20 ist in Fig. 3 näher dargestellt. Das Zwischenmodul 20 weist eine Kappe 30 zum Einführen und Schutz des Fingergliedes auf. Das Zwischenmodul 20 ist jeweils an einem proximalen Endbereich 32 mit dem Erstmodul 18 und an einem distalen Endbereich 34 mit dem Endmodul 22 gelenkig verbunden. Das Zwischenmodul 20 weist ferner Öffnungen 36 und hohle Stege 38 zur Führung der Schnüre auf.

Das Endmodul 22 ist in Fig. 4 näher dargestellt. Das Endmodul 22 weist einen komplementären Verbindungsabschnitt 40 für die lösbare Verbindung bzw. Kopplung mit der distalen Fingerprothesenmoduleinheit 14 auf. Der komplementäre Verbindungsabschnitt 40 ist hierbei ausgebildet eine lösbare formschlüssige Verbindung bzw. Steckverbindung mit der distalen Fingerprothesenmoduleinheit 14 einzugehen. Hierbei ist der komplementäre Verbindungsabschnitt 40 als nutenförmige Vertiefung 40 bzw. Sacklochnut 40 ausgebildet, in die ein nachfolgend näher beschriebener Vorsprung 58 der distalen Fingerprothesenmoduleinheit 14 entgegen einer bestimmungsgemäßen Griffrichtung 42 der distalen Fingerprothesenmoduleinheit 14 einschiebbar ist. Das Endmodul 22 weist ferner Öffnungen 44 an Stegen 46 zur distalen Fixierung der Schnüre auf.

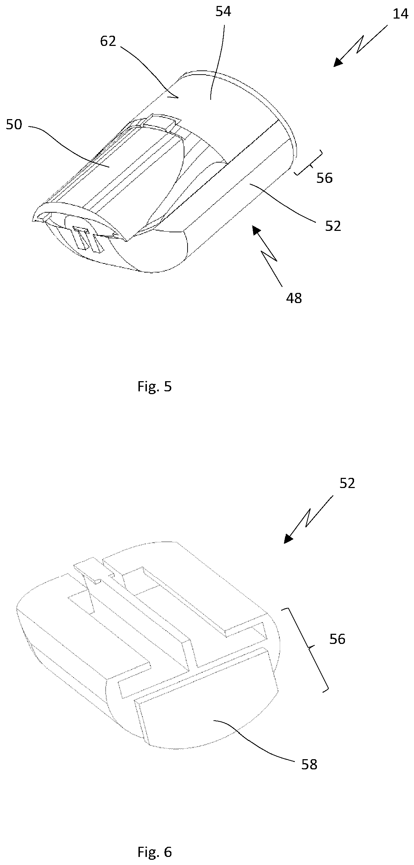

Die distale Fingerprothesenmoduleinheit 14 ist in Fig. 5 näher dargestellt. Die distale Fingerprothesenmoduleinheit 14 weist ein erfindungsgemäßes distales Fingerprothesenmodul 48 und eine erfindungsgemäße Funktionseinheit 50 auf.

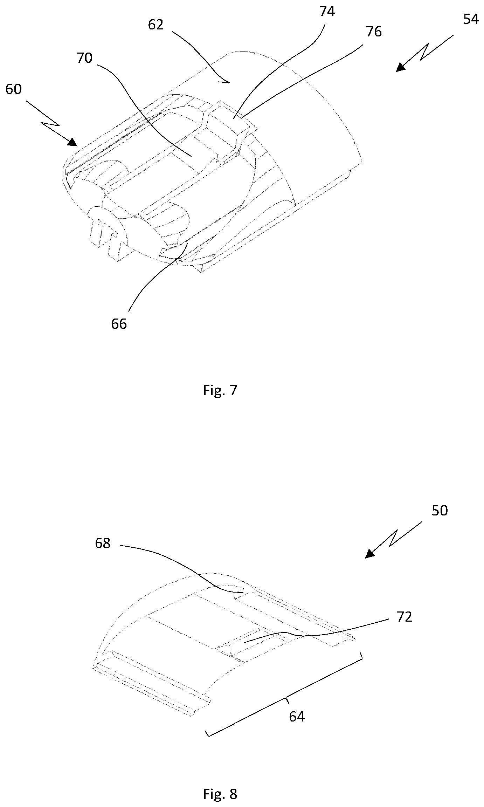

Das distale Fingerprothesenmodul 48 ist zweiteilig ausgebildet und weist ein in Fig. 6 näher dargestelltes Unterteil 52, welches als Fingerbeere fungiert, und ein in Fig. 7 näher dargestelltes Oberteil 54, welches als Nagelbett fungiert, auf. Das Unterteil 52 und das Oberteil 54 sind lösbar miteinander verbunden bzw. verbindbar.

Das distale Fingerprothesenmodul 48 weist zur lösbaren Verbindung mit dem proximalen Verbindungsmodul 12 bzw. dem komplementäre Verbindungsabschnitt 40 des Endmoduls 22 an einem proximalen Endbereich 56 das zuvor erwähnte Verbindungselement 58 auf, welches im gezeigten Ausführungsbeispiel an dem Unterteil 52 angeordnet ist. Das Verbindungselement 58 ist als Vorsprung 58 ausgebildet, welcher in die nutenförmige Vertiefung 40 des Endmoduls 22 entgegen der bestimmungsgemäßen Griffrichtung 42 einschiebbar ist.

Das distale Fingerprothesenmodul 48 weist ferner eine Aufnahmeeinheit 60 auf, welche ausgebildet ist, die Funktionseinheit 50 lösbar aufzunehmen. Die in Fig. 8 näher dargestellte Funktionseinheit 50 ist hierbei als künstlicher Nagel 50 ausgebildet und stellt eine zusätzliche technische Funktion bzw. ein zusätzliches Werkzeug für eine die modulare Fingerprothese 10 tragende Person bereit. Die Aufnahmeeinheit 60 ist an einer Oberseite 62 des Oberteils 54 bzw. des distalen Fingerprothesenmoduls 48 angeordnet.

Die Aufnahmeeinheit 60 ist ausgebildet, eine lösbare formschlüssige Verbindung mit einem komplementären Verbindungsabschnitt 64 der Funktionseinheit 50 bzw. des künstlichen Nagels 50 einzugehen. Hierfür weist die Aufnahmeeinheit 60 zwei nutenförmige Vertiefungen 66 an der Oberseite 62 auf, in die zwei entsprechend ausgebildete Vorsprünge 68 des komplementären Verbindungsabschnitts 64 der Funktionseinheit 50 proximal einschiebbar sind.

Die Aufnahmeeinheit 60 ist ferner ausgebildet, eine lösbare formschlüssige Rastverbindung mit der Funktionseinheit 50 einzugehen. Hierfür weist die Aufnahmeeinheit 60 einen elastisch federnden Rasthaken 70 auf, welcher in zumindest einer Rastvertiefung 72 des komplementären Verbindungsabschnitts 64 der Funktionseinheit 50 einrastbar ist.

Zur Freigabe der Rastverbindung weist die Aufnahmeeinheit 60 ein Entriegelungselement 74 auf, welches als Druckknopf 74 ausgebildet ist, und mittels dessen der elastisch federnde Rasthaken 70 betätigbar ist, um die eingerastete Funktionseinheit 50 bzw. den eingerasteten künstlichen Fingernagel 50 freizugeben. Das Entriegelungselement 74 bzw. der Druckknopf 74 ist hierbei an einem proximalen Endbereich 76 der Aufnahmeeinheit 60 angeordnet.

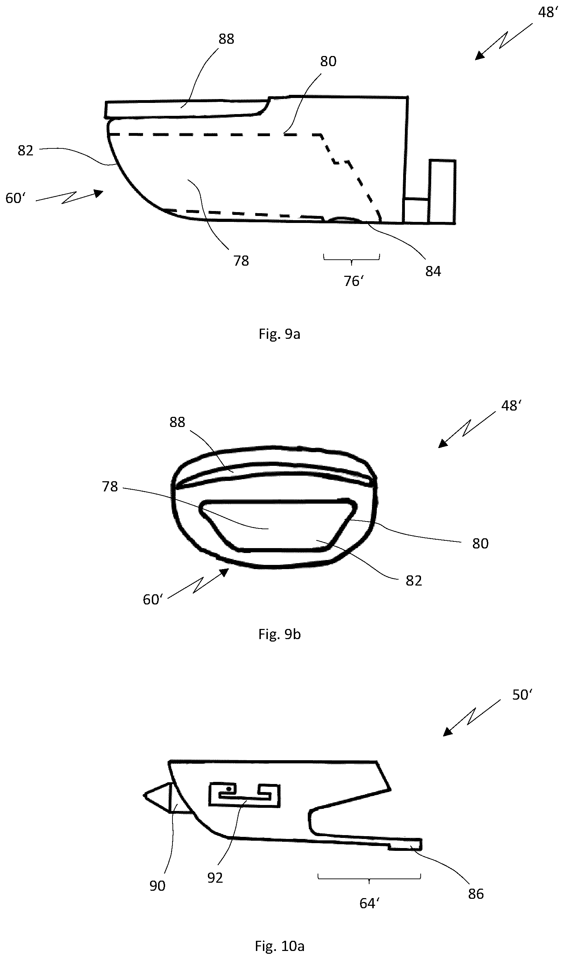

Fig. 9a und 9b zeigen ein weiteres Ausführungsbeispiel eines erfindungsgemäßen distalen Fingerprothesenmoduls 48' in einer Seitenansicht (Fig. 9a) und in einer Vorderansicht (Fig. 9b).

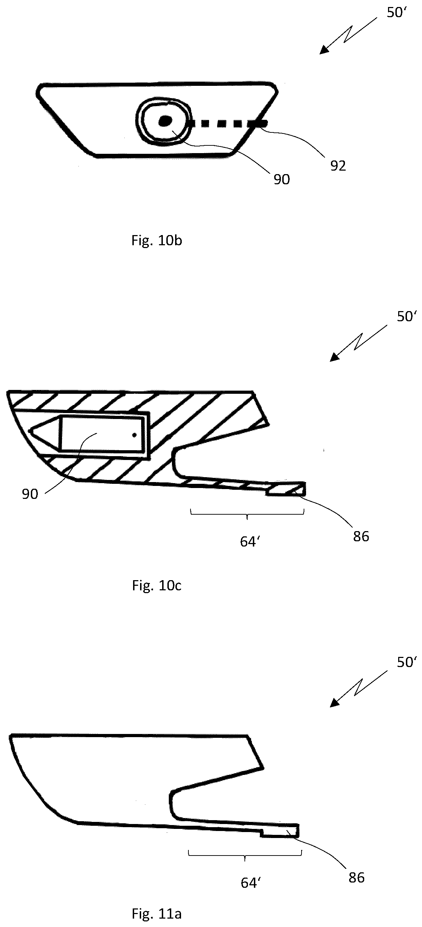

Das distale Fingerprothesenmodul 48' ist im Gegensatz zum Fingerprothesenmodul 48 aus Fig. 1 bzw. Fig. 5 einteilig ausgebildet. Ferner ist die Aufnahmeeinheit 60' des distalen Fingerprothesenmoduls 48' in einem Innenbereich 78 des distalen Fingerprothesenmoduls 48' angeordnet und ausgebildet, eine Funktionseinheit 50', welche in den Fig. 10 bis Fig. 13 näher dargestellt ist, lösbar aufzunehmen.

Die Aufnahmeeinheit 60' weist hierbei in dem Innenbereich 78 einen Aufnahmeraum 80 auf, in dem die Funktionseinheit 50' vollständig aufnehmbar ist, sodass eine lösbare formschlüssige Verbindung mit der Funktionseinheit 50' ausgebildet wird. Der Aufnahmeraum 80 weist eine Öffnung 82 zum Ein- und Ausbringen, insbesondere Ein- und Ausschieben der Funktionseinheit 50' auf.

Die Aufnahmeeinheit 60' ist ferner ausgebildet, eine lösbare formschlüssige Rastverbindung mit der Funktionseinheit 50' einzugehen. Hierfür weist die Aufnahmeeinheit 60' eine Rastvertiefung 84 auf, in die ein elastisch federnder Rasthaken 86 des komplementären Verbindungsabschnitts 64' der Funktionseinheit 50' einrastbar ist.

Zur Freigabe der Rastverbindung ist die Rastvertiefung 84 der Aufnahmeeinheit 60' derart ausgebildet, dass von außen Druck auf den eingerasteten elastisch federnden Rasthaken 86 der Funktionseinheit 50' ausübbar ist, um die eingerastete Funktionseinheit 50' freizugeben.

Die Rastvertiefung 84 ist hierbei an einem proximalen Endbereich 76' der Aufnahmeeinheit 60' angeordnet.

Hierbei sei angemerkt, dass der Nagel 88 nicht lösbar aufgenommen, sondern vielmehr fest mit dem distalen Fingerprothesenmodul 48' verbunden ist, sodass er im gezeigten Ausführungsbeispiel kein Teil der Funktionseinheit 50' ist. Es ist jedoch durchaus denkbar, dass das Fingerprothesenmodul 48' an der Oberseite gemäß dem Fingerprothesenmodul 48 aus Fig. 1 und 5 ausgebildet ist, sodass der Nagel 88 ebenfalls lösbar aufgenommen ist, ohne dabei den Rahmen der vorliegenden Erfindung zu verlassen.

Fig. 10 bis 13 zeigen diverse Ausführungsbeispiele erfindungsgemäßer Funktionseinheiten 50' für das distale Fingerprothesenmodul 48' aus Fig. 9a-b.

In Fig. 10a-c ist ein erstes Ausführungsbeispiel einer Funktionseinheit 50' in einer Seitenansicht (Fig. 10a), einer Vorderansicht (Fig. 10b) und einer Schnittdarstellung (Fig. 10c) gezeigt. Die Funktionseinheit 50' ist hierbei als Schreibeinheit 50' ausgebildet. Die Schreibeinheit 50' weist eine Schreibmine 90 auf, welche mittels eines Schiebers 92 aus dem Aufnahmeraum 80 ausschiebbar und in diesen wieder einschiebbar ist, um eine Schreibfunktion bereitzustellen.

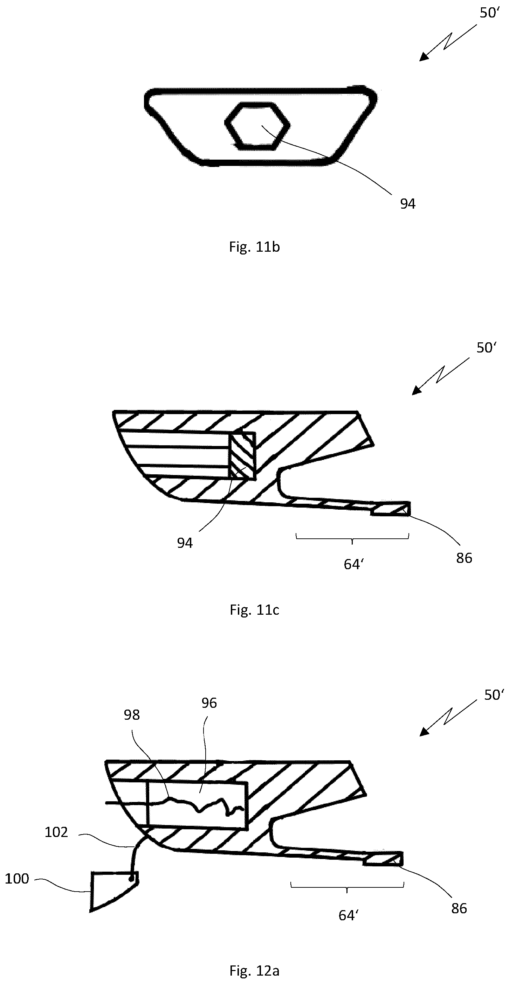

In Fig. 11a-c ist ein zweites Ausführungsbeispiel einer Funktionseinheit 50' in einer Seitenansicht (Fig. 11a), einer Vorderansicht (Fig. 11b) und einer Schnittdarstellung (Fig. 11c) gezeigt. Die Funktionseinheit 50' ist hierbei als Werkzeugeinheit 50' bzw. Werkzeughalter 50' ausgebildet. Der Werkzeughalter 50' weist einen Magneten 94 zum Halten eines (nicht gezeigten) entsprechend ausgebildeten Werkzeugs auf, welches bspw. als Schraubbit ausgeführt sein kann.

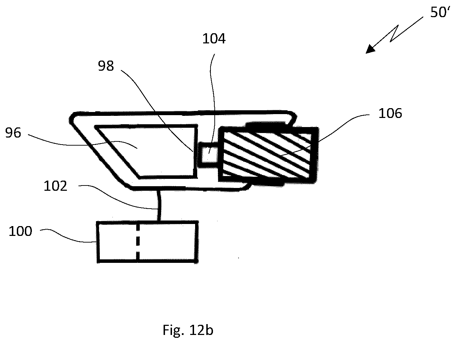

In Fig. 12a-b ist ein weiteres Ausführungsbeispiele einer Funktionseinheit 50' in einer Schnittdarstellung (Fig. 12a) und in einer seitliche Detailansicht (Fig. 12b) gezeigt. Die Funktionseinheit 50' ist hierbei als Erwärmungseinheit 50' bzw. Feuerzeug 50' ausgebildet. Das Feuerzeug 50' weist eine Benzinkammer 96, einen Docht 98 sowie einen Deckel 100, welcher über ein flexibles Band 102 angebunden ist, auf. Wie aus der Detailansicht von Fig. 12b ersichtlich, weist das Feuerzeug 50' ferner einen Zündstein 104 und ein Reibrad 106 auf.

Umfasst ein Ausführungsbeispiel eine "und/oder"-Verknüpfung zwischen einem ersten Merkmal und einem zweiten Merkmal. So ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Distale Fingerprothesenmoduleinheit (14) für eine modulare Fingerprothese (10) mit
- einer Funktionseinheit (50') und mit einem als distales Fingerprothesenglied ausgebildeten distalen Fingerprothesenmodul (48') aufweisend ein Verbindungselement (58) zur lösbaren Verbindung des Fingerprothesenmoduls (48') mit der modularen Fingerprothese (10), und aufweisend eine Aufnahmeeinheit (60') zur lösbaren Aufnahme der Funktionseinheit (50'), **dadurch gekennzeichnet, dass** die Funktionseinheit zur Bereitstellung einer zur Grifffunktion des distalen Fingerprothesenmoduls (48') zusätzlichen Funktion für eine die modulare Fingerprothese (10) tragende Person, ausgebildet ist und
- die Funktionseinheit in in der Aufnahmeeinheit (60') des distalen Fingerprothesenmoduls (48') lösbar aufgenommen ist, wobei die Aufnahmeeinheit (60') zur lösbaren Aufnahme der Funktionseinheit (50'), insbesondere zur lösbaren formschlüssigen Verbindung mit der Funktionseinheit (50') zumindest einen Aufnahmeraum (80) in einem Innenbereich (78) des distalen Fingerprothesenmoduls (48') aufweist, in den die Funktionseinheit (50'), insbesondere vollständig aufgenommen ist.

2. Distale Fingerprothesenmoduleinheit (14) nach Anspruch 1, **dadurch gekennzeichnet, dass** der Aufnahmeraum (80) eine, insbesondere schließbare Öffnung (82) zum Ein- und Ausbringen, insbesondere Ein- und Ausschieben der Funktionseinheit (50') aufweist.

3. Distale Fingerprothesenmoduleinheit (14) nach Anspruch 1 oder 2, **dadurch gekennzeichnet, dass** die Aufnahmeeinheit (60') eine lösbare formschlüssige Verbindung, insbesondere eine lösbare formschlüssige Rastverbindung mit einem komplementären Verbindungsabschnitt (64') der Funktionseinheit (50') eingeht.

4. Distale Fingerprothesenmoduleinheit (14) nach Anspruch 3, **dadurch gekennzeichnet, dass** die Aufnahmeeinheit (60') zur lösbaren formschlüssigen Rastverbindung mit der Funktionseinheit (50') zumindest eine Rastvertiefung (84) aufweist, in die zumindest ein elastisch federnder Rasthaken (86) des komplementären Verbindungsabschnitts (64') der Funktionseinheit (50') eingerastet ist.

5. Distale Fingerprothesenmoduleinheit (14) nach Anspruch 4, **dadurch gekennzeichnet, dass** zur Freigabe der Rastverbindung die zumindest eine Rastvertiefung (84) derart ausgebildet ist, dass von außen Druck auf den eingerasteten elastisch federnden Rasthaken (86) der Funktionseinheit (50') ausübbar ist, um die eingerastete Funktionseinheit (50') freizugeben, insbesondere wobei die Rastvertiefung (84) an einem proximalen Endbereich (76') der Aufnahmeeinheit (60') angeordnet ist.

6. Distale Fingerprothesenmoduleinheit (14) nach einem der vorhergehenden Ansprüche, **dadurch gekennzeichnet, dass** das Verbindungselement (58) ausgebildet ist, eine lösbare formschlüssige Verbindung, insbesondere lösbare formschlüssige Rastverbindung mit einem komplementären Verbindungsabschnitt (40) der modularen Fingerprothese (10) einzugeben.

7. Distale Fingerprothesenmoduleinheit (14) nach Anspruch 6, **dadurch gekennzeichnet, dass** das Verbindungselement (58) zur lösbaren formschlüssigen Verbindung mit der modularen Fingerprothese (10)
- zumindest einen Vorsprung (58) aufweist, welcher in zumindest eine nutenförmige Vertiefung (40) des komplementären Verbindungsabschnitts (40) der modularen Fingerprothese (10), insbesondere entgegen einer bestimmungsgemäßen Griffrichtung (42) des distalen Fingerprothesenmoduls (48') einschiebbar ist, und/oder
- zumindest eine nutenförmige Vertiefung aufweist, in die zumindest ein Vorsprung des komplementären Verbindungsabschnitts (40) der modularen Fingerprothese (10), insbesondere entgegen einer/der bestimmungsgemäßen Griffrichtung (42) des distalen Fingerprothesenmoduls (48') einschiebbar ist.

8. Distale Fingerprothesenmoduleinheit (14) nach einem der vorhergehenden Ansprüche, **dadurch gekennzeichnet, dass** die Funktionseinheit (50') zumindest eine der folgenden Einheiten aufweist: Lichteinheit, insbesondere LED-Beleuchtungseinheit und/oder Laserpointer; Datenspeichereinheit, insbesondere USB-Stick; Energiespeichereinheit, insbesondere Powerbank; Erwärmungseinheit (50'), insbesondere Feuerzeug (50'); Schreibeinheit (50'), insbesondere Minenhalter für eine Schreibmine (90) oder mit einer Schreibmine (90); Werkzeugeinheit (50'), insbesondere Werkzeughalter (50') für ein Werkzeug und/oder mit einem Werkzeug; Magneteinheit, insbesondere für ein berührungsempfindliches Tastfeld; Zwille; Zerstäubungseinheit, insbesondere Sprüheinheit; Identifikationseinheit, insbesondere RFID-Chip.

9. Modulare Fingerprothese (10) mit einer distalen Fingerprothesenmoduleinheit (14) nach einem der vorhergehenden Ansprüche, welche mittels des Verbindungselements (58) mit der modularen Fingerprothese (10) lösbar verbunden ist.

## Claims

1. A distal finger prosthesis module unit (14) for a modular finger prosthesis (10), comprising:
a functional unit (50'); and a distal finger prosthesis module (48') configured as a distal finger prosthesis member, having a connecting element (58) for detachably connecting the finger prosthesis module (48') to the modular finger prosthesis (10), and having a receiving unit (60') for detachably receiving the functional unit (50'); **characterized in that the** functional unit (50') is configured to provide an additional function, relative to a gripping function of the distal finger prosthesis module (48'), for a person wearing the modular finger prosthesis (10), and the functional unit (50') is detachably received in the receiving unit (60') of the distal finger prosthesis module (48'), wherein the receiving unit (60') comprises, for detachably receiving the functional unit (50'), in particular for a detachable positive-fit connection with the functional unit (50'), at least one receiving space (80) in an interior region (78) of the distal finger prosthesis module (48'), in which receiving space the functional unit (50') is received, in particular completely received.

2. The distal finger prosthesis module unit (14) according to claim 1, **characterized in that** the receiving space (80) has an opening (82), in particular a closable opening (82), for inserting and removing, in particular for sliding in and sliding out, the functional unit (50').

3. The distal finger prosthesis module unit (14) according to claim 1 or 2, **characterized in that** the receiving unit (60') forms a detachable positive-fit connection, in particular a detachable positive-fit snap-in connection, with a complementary connecting section (64') of the functional unit (50').

4. The distal finger prosthesis module unit (14) according to claim 3, **characterized in that** the receiving unit (60') comprises at least one snap-in recess (84) for the detachable positive-fit snap-in connection with the functional unit (50'), into which at least one elastically resilient snap-in hook (86) of the complementary connecting section (64') of the functional unit (50') is engaged.

5. The distal finger prosthesis module unit (14) according to claim 4, **characterized in that**, for releasing the snap-in connection, the at least one snap-in recess (84) is configured such that pressure can be exerted from the outside on the engaged elastically resilient snap-in hook (86) of the functional unit (50') in order to release the engaged functional unit (50'), in particular wherein the snap-in recess (84) is arranged at a proximal end region (76') of the receiving unit (60').

6. The distal finger prosthesis module unit (14) according to any one of the preceding claims, **characterized in that** the connecting element (58) is configured to enter into a detachable positive-fit connection, in particular a detachable positive-fit snap-in connection, with a complementary connecting section (40) of the modular finger prosthesis (10).

7. The distal finger prosthesis module unit (14) according to claim 6, **characterized in that** the connecting element (58) for the detachable positive-fit connection with the modular finger prosthesis (10) comprises
- at least one projection (58) which can be inserted into at least one groove-shaped recess (40) of the complementary connecting section (40) of the modular finger prosthesis (10), in particular counter to an intended gripping direction (42) of the distal finger prosthesis module (48'); and/or
- at least one groove-shaped recess into which at least one projection of the complementary connecting section (40) of the modular finger prosthesis (10) can be inserted, in particular counter to an/the intended gripping direction (42) of the distal finger prosthesis module (48').

8. The distal finger prosthesis module unit (14) according to any one of the preceding claims, **characterized in that** the functional unit (50') comprises at least one of the following units: light unit, in particular LED lighting unit and/or laser pointer; data storage unit, in particular USB stick; energy storage unit, in particular power bank; heating unit (50'), in particular lighter (50'); writing unit (50'), in particular refill holder for a writing refill (90) or with a writing refill (90); tool unit (50'), in particular tool holder (50') for a tool and/or with a tool; magnet unit, in particular for a touch-sensitive touchpad; slingshot; atomizing unit, in particular spraying unit; identification unit, in particular RFID chip.

9. A modular finger prosthesis (10) comprising a distal finger prosthesis module unit (14) according to any one of the preceding claims, which is detachably connected to the modular finger prosthesis (10) by means of the connecting element (58).

## Revendications

1. Unité de module de prothèse de doigt distale (14) pour une prothèse de doigt modulaire (10), comprenant:
une unité fonctionnelle (50') ; et un module de prothèse de doigt distal (48') conçu sous la forme d'un élément de prothèse de doigt distal, présentant un élément de liaison (58) pour la liaison amovible du module de prothèse de doigt (48') à la prothèse de doigt modulaire (10), et présentant une unité de logement (60') pour le logement amovible de l'unité fonctionnelle (50') ; **caractérisée en ce que** l'unité fonctionnelle (50') est conçue pour fournir une fonction supplémentaire à une fonction de préhension du module de prothèse de doigt distal (48') pour une personne portant la prothèse de doigt modulaire (10), et l'unité fonctionnelle (50') est logée de manière amovible dans l'unité de logement (60') du module de prothèse de doigt distal (48'), dans laquelle l'unité de logement (60') présente, pour le logement amovible de l'unité fonctionnelle (50'), en particulier pour une liaison amovible par complémentarité de forme avec l'unité fonctionnelle (50'), au moins un espace de logement (80) dans une zone interne (78) du module de prothèse de doigt distal (48'), dans lequel l'unité fonctionnelle (50') est logée, en particulier complètement logée.

2. Unité de module de prothèse de doigt distale (14) selon la revendication 1, **caractérisée en ce que** l'espace de logement (80) présente une ouverture (82), en particulier une ouverture fermable (82), pour l'introduction et l'extraction, en particulier l'insertion et l'extraction par coulissement, de l'unité fonctionnelle (50').

3. Unité de module de prothèse de doigt distale (14) selon la revendication 1 ou 2, **caractérisée en ce que** l'unité de logement (60') établit une liaison amovible par complémentarité de forme, en particulier une liaison amovible par encliquetage par complémentarité de forme, avec une section de liaison complémentaire (64') de l'unité fonctionnelle (50').

4. Unité de module de prothèse de doigt distale (14) selon la revendication 3, **caractérisée en ce que** l'unité de logement (60') présente, pour la liaison amovible par encliquetage par complémentarité de forme avec l'unité fonctionnelle (50'), au moins un évidement d'encliquetage (84) dans lequel est encliqueté au moins un crochet d'encliquetage élastiquement flexible (86) de la section de liaison complémentaire (64') de l'unité fonctionnelle (50').

5. Unité de module de prothèse de doigt distale (14) selon la revendication 4, **caractérisée en ce que**, pour libérer la liaison par encliquetage, le au moins un évidement d'encliquetage (84) est conçu de telle sorte qu'une pression peut être exercée depuis l'extérieur sur le crochet d'encliquetage élastiquement flexible (86) encliqueté de l'unité fonctionnelle (50') afin de libérer l'unité fonctionnelle (50') encliquetée, en particulier dans laquelle l'évidement d'encliquetage (84) est disposé au niveau d'une zone d'extrémité proximale (76') de l'unité de logement (60').

6. Unité de module de prothèse de doigt distale (14) selon l'une quelconque des revendications précédentes, **caractérisée en ce que** l'élément de liaison (58) est conçu pour établir une liaison amovible par complémentarité de forme, en particulier une liaison amovible par encliquetage par complémentarité de forme, avec une section de liaison complémentaire (40) de la prothèse de doigt modulaire (10).

7. Unité de module de prothèse de doigt distale (14) selon la revendication 6, **caractérisée en ce que** l'élément de liaison (58), pour la liaison amovible par complémentarité de forme avec la prothèse de doigt modulaire (10) présente
- au moins une saillie (58) qui peut être insérée dans au moins un évidement en forme de rainure (40) de la section de liaison complémentaire (40) de la prothèse de doigt modulaire (10), en particulier à l'encontre d'une direction de préhension prévue (42) du module de prothèse de doigt distal (48') ; et/ou
- au moins un évidement en forme de rainure dans lequel au moins une saillie de la section de liaison complémentaire (40) de la prothèse de doigt modulaire (10) peut être insérée, en particulier à l'encontre d'une/de la direction de préhension prévue (42) du module de prothèse de doigt distal (48').

8. Unité de module de prothèse de doigt distale (14) selon l'une quelconque des revendications précédentes, **caractérisée en ce que** l'unité fonctionnelle (50') présente au moins l'une des unités suivantes : unité d'éclairage, en particulier unité d'éclairage LED et/ou pointeur laser ; unité de stockage de données, en particulier clé USB ; unité de stockage d'énergie, en particulier batterie externe (powerbank) ; unité de chauffage (50'), en particulier briquet (50') ; unité d'écriture (50'), en particulier porte-mine/porte-recharge pour une recharge d'écriture (90) ou avec une recharge d'écriture (90) ; unité d'outil (50'), en particulier porte-outil (50') pour un outil et/ou avec un outil ; unité magnétique, en particulier pour un pavé tactile sensible au toucher ; lance-pierre ; unité d'atomisation, en particulier unité de pulvérisation ; unité d'identification, en particulier puce RFID.

9. Prothèse de doigt modulaire (10) comprenant une unité de module de prothèse de doigt distale (14) selon l'une quelconque des revendications précédentes, qui est reliée de manière amovible à la prothèse de doigt modulaire (10) au moyen de l'élément de liaison (58).
